# EUROPEAN PATENT APPLICATION

(11) **EP 3 838 115 A1**
(43) Date of publication of application: **23.06.2021**
(21) Application number: 19217834.1
(22) Date of filing: 19.12.2019
(51) Int. Cl.: A61B 5/00, A61B 5/024, A61B 5/1455, A61B 5/11, A61B 5/113

(54) **DEVICE, SYSTEM AND METHOD FOR DETERMINING A VITAL SIGN OF A SUBJECT**

(71) Applicant: Koninklijke Philips N.V., 5656 AG Eindhoven (NL)
(72) Inventor: VERKRUIJSSE, Willem, 5656 AE Eindhoven (NL); VAREKAMP, Christiaan, 5656 AE Eindhoven (NL)
(74) Representative: Philips Intellectual Property & Standards

(57) **Abstract**

The present invention relates to a device (100) for determining a vital sign of a subject by use of PPG. The device comprises a processing unit (110) configured to obtain multiple first detection signals derived from electromagnetic radiation (90) from multiple measurement spots (30) on a skin region (12) of the subject illuminated by one or more illumination spots (20), wherein the electromagnetic radiation (90) enters the skin region (12) at the one or more illumination spots (20) and exits the skin region (12) at the multiple measurement spots (30). The processing unit (110) further obtains 3D image information (130) of the skin region (12) which allows determining the actual spot distance (15) between the measurements spots (30) and an illumination spot (20), respectively. This information can be used to interpret the PPG signal more precisely as without having the 3D image information only a projected distance can be determined if the skin surface is tilted. Additionally, this allows averaging PPG signals derived from a homogenous skin area to improve PPG signal strength and reduce noise. A system (500) comprising such a device and a corresponding method are further described.

## Description

### FIELD OF THE INVENTION

The present invention relates to a device, system and method for determining a vital sign of a subject.

### BACKGROUND OF THE INVENTION

Vital signs of a person, for example the heart rate, the respiration rate or the arterial blood oxygen saturation, serve as indicators of the current state of a person and as powerful predictors of serious medical events. For this reason, vital signs are extensively monitored in inpatients and outpatients care settings, at home or in further health, leisure and fitness settings.

One way of measuring vital signs is plethysmography (PPG). PPG generally refers to the measurement of volume changes of an organ or a body part and in particular to the detection of volume changes due to a cardio vascular pulse wave traveling through the body of a subject with every heart beat.

To be more concrete, PPG is an optical measurement technique that evaluates a time variant change of light reflectance or transmission of an area or volume of interest and is based on the principle that blood absorbs light more than surrounding tissue. Hence, variations in blood volume with every heart beat effect transmission or reflectance correspondingly. Besides information about the heart rate, a PPG waveform can comprise information attributable to further physiological phenomena such as the respiration. By evaluating the transmittance and/or reflectivity at different wavelengths (typically red and infrared), the blood oxygen saturation, SpO2, can even be determined.

Recently, non-contact remote PPG (rPPG) devices for unobtrusive measurements have been introduced. Remote PPG utilizes light sources or, in general radiation sources, disposed remotely from the subject of interest. Similarly, also a detector, e.g., a camera or a photodetector, can be disposed remotely from the subject of interest. Contactless camera based PPG as a special kind of non-contact remote PPG typically uses widefield (homogeneous) illumination and detection. Hence, this technique is referred to as "*widefield PPG*" in the following. PPG signal strengths in widefield PPG are typically very small (less than 1 % of modulation), because most of the backscattered/remitted light has traveled only very short distances through the skin of the subject. This very small signal strength is a significant disadvantage of contactless camera based PPG compared to conventional contact PPG probes (with at least a factor of 10-100 difference). On many anatomical locations, the widefield PPG signal is in fact unmeasurable (e.g., on the leg or on the arm) while with contact sensors this is possible.

Another possibility of contactless camera based PPG is based on the use of illumination patterns or based on the use of just a single spot created, e.g., by a laser and is proposed and referred to herein as "*radial PPG*" in the following. An advantage of radial PPG is that it is possible to measure light that has travelled larger distances through the skin than for widefield PPG. As a result, significantly larger PPG modulation depths can be measured, which enables to overcome the disadvantages of typical widefield camera based PPG discussed above. A disadvantage is, however, that the spatial resolution is decreased compared to widefield PPG. Another problem arising from radial PPG is that if only one illumination spot is evaluated, just a few camera pixels have a remitting signal in the dynamic range of the camera. Further, the scale of the image is uncalibrated, which does not allow meaningful evaluation of the radial PPG signal.

### SUMMARY OF THE INVENTION

Based on the different problems arising from conventional radial PPG it is an object of the present invention to provide a more accurate device, method and system for determining a vital sign of a subject.

In a first aspect of the present invention, a device for determining a vital sign of a subject is presented that comprises a processing unit configured to:
- obtain multiple first detection signals derived from electromagnetic radiation from multiple measurement spots on a skin region of the subject illuminated by one or more illumination spots, wherein the electromagnetic radiation enters the skin region at the one or more illumination spots and exits the skin region at the multiple measurement spots,
- obtain a 3D image information of the skin region,
- determine for the multiple first detection signals the respective spot distance between the respective measurement spot and an illumination spot based on the obtained 3D image information of the skin region,
- assign the multiple first detection signals to their respective spot distance and to the illumination spot from which the respective spot distance is determined, and
- average the first detection signals assigned to the same illumination spot and to a respective spot distance smaller than a threshold distance value to obtain an averaged first detection signal, and determine an averaged vital sign from said averaged first detection signal, or
- determine vital signs from the first detection signals assigned to the same illumination spot and to a respective spot distance smaller than a threshold distance value, and average said vital signs to obtain an averaged vital sign.

According to another aspect of the present invention, a system for determining a vital sign of a subject is presented that comprises, besides the above-described device, an illumination unit configured to emit electromagnetic radiation to illuminate a skin region of the subject by the one or more illumination spots, and a camera configured to record an image of the detected electromagnetic radiation reflected from the skin region illuminated by one or more illumination spots and to derive multiple first detection signals from the recorded images.

In yet further aspects of the present invention, there are provided a corresponding method, a computer program which comprises program code means for causing a computer to perform the steps of the method disclosed herein when said computer program is carried out on a computer as well as a non-transitory computer readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea that radial PPG measurements with an illumination by one or more illumination spots (e.g., emitted by a laser), require knowledge about the actual spot distance of the measurement spot from which the PPG signal is derived to the illumination spot(s) on the skin region. In contrast to widefield PPG, where the skin of a subject is illuminated homogenously and the widefield PPG signal strength is independent from the position on the skin and the local orientation of the skin, the radial PPG signal can depend strongly on both position (relative to illuminated parts) and skin surface orientation.

If the skin surface orientation is perpendicular to the illumination direction and perpendicular to the recording camera, it is relatively straightforward to measure the absolute spot distance with a camera, since lateral distances in the images may be easily calibrated (e.g., through known angular distances between the illumination dot(s) and the measurement dots). For curved skin regions, it requires correction for projection, and the determination of the actual spot distance is much more complex. It shall be understood that a skin surface orientation perpendicular to the recording camera means in said context that the skin surface points with its full surface to the recording camera.

For this purpose, the processing unit of the claimed device is configured to obtain 3D image information of the skin region. This allows determining for the multiple first detection signals, derived from electromagnetic radiation from multiple measurements spots located on the skin region, the respective spot distance between the respective measurement spot and an illumination spot based on the obtained 3D image information of the skin region. This is of particular interest, as mentioned above, for curved skin regions. Without obtaining the 3D image information of the skin region, determining the actual respective spot distances would not be possible as only projected distances may be determined.

According to the principles of the present invention, with having knowledge about the actual spot distances, the multiple first detection signals are assigned to the respective spot distance and to the illumination spot from which the respective spot distance is determined. This may be done by the processing unit, which obtains the first detection signals derived from images recorded by the camera. Hence, the processing unit may thus be configured to extract from said recorded images information about the location of the multiple measurement spots and the location of the illumination spot(s) by any kind of image recognition known in the art, such as Statistical Techniques, Structural Techniques, Template Matching, Neural Network Approach, Fuzzy Model and Hybrid Models to determine the respective distances there between.

In said context, the respective spot distance is the distance between the measurement spot from which the respective first detection signal is derived and an illumination spot (of the multiple illumination spots, if a spot pattern is used for illumination). This further allows averaging the first detection signals assigned to the same illumination spot and to a respective spot distance smaller than a threshold distance value to reduce the noise level by averaging PPG signals derived from a homogenous skin area. This threshold distance value may be arbitrary and set by the processing unit or manually by a user depending on the location on the skin region of the subject from which the PPG signals are measured. Typical values are in a range of some millimeters up to some centimeters.

Averaging the multiple signals allows reducing the high noise inherently associated with radial PPG by averaging multiple pixels (from multiple measurement spots) with substantially equal distance to an illumination spot in a given area. This is of particular interest for radial PPG as even though the relative pulsatile signal (AC/DC) in radial PPG may be larger, the DC components can be very small and thus very noisy. This is because of two reasons: Firstly, because averaging takes place of fewer pixels compared to widefield PPG where typically a larger skin area is homogenously illuminated, and secondly, because the DC signal is measured away from the illumination spot, and is thus much weaker as lots of signal strength is absorbed by the skin.

According to an embodiment, the processing unit may be further configured to derive a first detection function from the first detection signals assigned to the same illumination spot, wherein the first detection function is a function of the spot distance. As mentioned above, using the 3D image information of the skin region allows determining the actual spot distances, rather than the projected spot distances. Hence, knowledge about the actual distances on the skin region with respect to the illumination spot is determined. This allows deriving a first detection function, which is a function of said lateral distances away from an illumination spot. Analyzing said first detection function and further processing of the first detection signals (e.g., by calculating the respective vital signs or just by determining the respective relative PPG amplitudes) allows obtaining a spatial resolution of the physiological signals. This may be used to define, e.g., a color map, which represents the PPG amplitudes for different skin pixels of the measured skin area. Thus, an improved color map may be obtained, which gives clinicians further support and may increase the acceptance of PPG imaging in medical settings.

According to another embodiment, the processing unit may be configured to determine the 3D information of the skin region from the multiple first detection signals in case the skin region is illuminated by multiple illumination spots. Preferably, the light emitted by the illumination unit to illuminate the subject is infrared light. Structured infrared light projection is a commonly known technique for depth imaging. If the processing unit is configured on its own to determine the 3D information of the skin region from the multiple first detection signals, an external sensor for obtaining 3D depth information (such as a time-of-flight (TOF) sensor) is not needed. Thus, a compact device is presented that allows determining more accurate vital signs by being configured on its own to determine depth information. The device may thus be a compact, portable device, which may not only be used in stationary clinical settings, but further, e.g., for ambulant patients.

According to another embodiment, the processing unit may, in the case of multiple illumination spots, configured to determine for the multiple first detection signals the respective spot distance between the respective measurement spot and the illumination spot, which is the closest illumination spot to the respective measurement spot in relation to the other illumination spots. Hence, only the respective distances between measurement spots and illumination spots which are close to each other have to be considered for determining the respective distances there between. This reduces processing time to quickly determine the respective spot distances and assign all measurement spots to a respective illumination spot.

According to another embodiment, the processing unit may be configured to
- obtain a second detection signal derived from a spatial integral of all the electromagnetic radiation reflected from the skin region of the subject illuminated by one or more illumination spots or derived from electromagnetic radiation reflected from or transmitted through skin region of the subject illuminated by a homogenous illumination, and
- determine one or more normalized signals, wherein a normalized signal is calculated by calculating a ratio of a first detection signal of the multiple first detection signals and the second detection signal.

Preferably, this second detection signal is also derived from the image(s) recorded by the camera. The second detection signal may thus be derived from a spatial integral of the electromagnetic radiation reflected from all illuminated skin pixels. The second detection signal is a widefield PPG signal. Widefield PPG is typically measured by using a light source that emits electromagnetic radiation homogenously onto a skin region of a subject. However, using structured light, i.e., a pattern of dots, stripes, circles, etc. works as well. In fact, any non-homogenous illumination pattern can work, albeit with great deconvolution work. Further, even using just a spot illumination (i.e., one laser spot on the skin of the subject) and determining the spatial integral of all light reflected back from the skin of the subject is also suitable for providing widefield PPG.

The normalized signals are determined by calculating a ratio of a first detection signal of the multiple first detection signals (radial PPG signals) and the second detection signal (widefield PPG signal). Preferably, the first detection signals and the second detection signal are derived from electromagnetic radiation with the same wavelength, such as exemplarily electromagnetic radiation in the near-infrared spectral range. Hence, the normalized signals may indicate signals relating to electromagnetic radiation in the near-infrared spectral range. In said context, the first normalized signals are a measure for the penetration depth of electromagnetic radiation in the near-infrared spectral range in the skin of the subject as the ratio of a radial PPG signal and a widefield PPG signal allows obtaining an estimate for the depth of pulsatile layers in the skin region. This is highly advantageous in comparison to conventional PPG imaging, which constantly has the problem of ambiguity. To be more concrete, the actual PPG amplitude is difficult to interpret for conventional PPG imaging as it is the integral of PPG modulations stemming from different skin depths. Superficial vessels with a very small pulsatility may, for example, give the same PPG signal strength as deeper vessels with a stronger pulsatility.

By determining the normalized signals as disclosed herein, the depth of the pulsatile vessels may be estimated. This allows interpreting PPG images much more precisely with less ambiguity.

The above-mentioned system according to the present invention comprises an illumination unit configured to emit electromagnetic radiation to illuminate the skin region of the subject by one or more illumination spots. These illumination spots may have the shape of dots, circles, ellipses or stripes. Thus, in said context the term "spots" is not limited to any kind of shape. It should be noted that circles have several benefits as the number of pixels that are in the dynamic range of the camera, which records an image of the detected electromagnetic radiation reflected from the skin region, is increased in comparison to other illumination profiles, such as dots.

The system may further comprise a time-of-flight (TOF) sensor to determine 3D image information of the skin region from the detected electromagnetic radiation reflected from the skin region illuminated by one or more illumination spots and/or a stereo depth sensor to determine 3D image information by stereo vision. Another option may be to assume a 3D skin model (such as a 3D face model), to estimate the pose of the 3D face and to use this as 3D image information. Another option may be to combine the various different sensors, such as a TOF sensor and a stereo depth sensor, in one combined sensor approach.

As mentioned above, the processing unit of the device may be configured to determine 3D image information of the skin region from the multiple first detection signals in case the skin region is illuminated by multiple illumination spots. If the skin region is only illuminated by one illumination spot, it may be an option to use a TOF sensor and/or a stereo depth sensor to obtain the 3D image information needed to determine more accurate vital signs. An advantage of a stereo depth sensor is that in contrast to other sensors for depth detection, such as time-of-flight cameras or structured light, stereo vision is a purely passive process and can therefore also be used outdoors in daylight. This allows providing accurate 3D perception even under difficult conditions such as bright daylight, long distance measurements or overlapping ranges.

According to an embodiment, the system may further comprise a control unit, which is preferably connected to the illumination unit and to the camera. In the case where the illumination unit is configured to emit electromagnetic radiation by one or more illumination spots having the shape of a circle or an ellipse, said control unit is configured to receive 3D image information of the skin region and a color image of the illuminated skin region recorded by the camera being a color camera. The 3D depth information may be received from the processing unit of the device in an embodiment, where said processing unit is configured to determine 3D image information on its own, or from the TOF sensor and/or from the stereo depth sensor. Based on the received 3D image information and the color image, the control unit may control the shape of the one or more illumination spots emitted by the illumination unit to emit one or more illumination spots having the shape of such an ellipse that the one or more illumination spots have the shape of a circle on the skin region in the color image recorded by the color camera. This may be done by using any suitable kind of image recognition by the control unit to recognize the shape of the ellipse in the received recorded image and to adapt the shape of the emitted ellipse based thereon.

This all is of particular interest if the skin surface is not perpendicular to the illumination direction as in this case a circle projected onto the skin surface may result in a shape of an ellipse in the image recorded by the camera. The shape of an ellipse is, however, not desired according to some embodiments. An ellipse leads to a much more complicated post-processing of the obtained signals as the circular symmetry is broken. Thus, the control unit may control the illumination of the illumination unit such that the recorded image of the camera comprises circular spots again.

### BRIEF DESCRIPTION OF THE DRAWINGS

These and other aspects of the invention will be apparent from and elucidated with reference to the embodiments described hereinafter. In the following drawings
Figs. 1A and 1B show schematic diagrams illustrating the paths of electromagnetic radiation through skin region of a subject for widefield PPG and radial PPG;
Figs. 2A and 2B show schematic diagrams illustrating the detected reflected light of widefield PPG and radial PPG;
Fig. 3 shows a schematic diagram of Monte Carlo simulations of the paths of light through a skin region of a subject for radial PPG;
Figs. 4A and 4B show schematic diagrams illustrating the path of electromagnetic radiation through skin region of a subject for radial PPG;
Figs. 5A-5C show schematic diagrams illustrating ambiguity problems arising for conventional PPG;
Fig. 6 shows a system for determining a vital sign of a subject according to the present invention;
Fig. 7 shows a modified embodiment of the system shown in Fig. 6 in order to illuminate a skin region homogenously;
Fig. 8 shows a flowchart illustrating a method to be executed by the device according to the present invention;
Fig. 9 shows a schematic diagram illustrating problems arising from radial PPG without having knowledge about the 3D structure of a measured skin region;
Fig. 10 shows an infrared image and a 3D depth image of a mannequin;
Fig. 11 shows a flowchart illustrating an optional method to be executed by the device according to the present invention;
Figs. 12A and 12B show schematic diagrams illustrating problems arising from conventional radial PPG without having knowledge about the 3D structure of a measured skin region;
Fig. 13 shows a schematic diagram illustrating an area illuminated by a spot pattern;
Figs. 14A and 14B show a schematic diagrams illustrating a further possibility of reducing PPG noise;
Figs. 15A-15C show schematic diagrams illustrating the projection of a pattern of circles on a skin region of a subject;
Fig. 16 shows a schematic diagram illustrating a comparison of an illumination with a dot pattern and an illumination with a circular pattern;
Fig. 17 shows a schematic diagram illustrating locally measured PPG amplitudes;
Fig. 18 shows a processing of radial PPG signals and widefield PPG signals to overcome problems arising from conventional PPG; and
Fig. 19 shows a schematic diagram illustrating a process of obtaining improved PPG color maps.

### DETAILED DESCRIPTION OF EMBODIMENTS

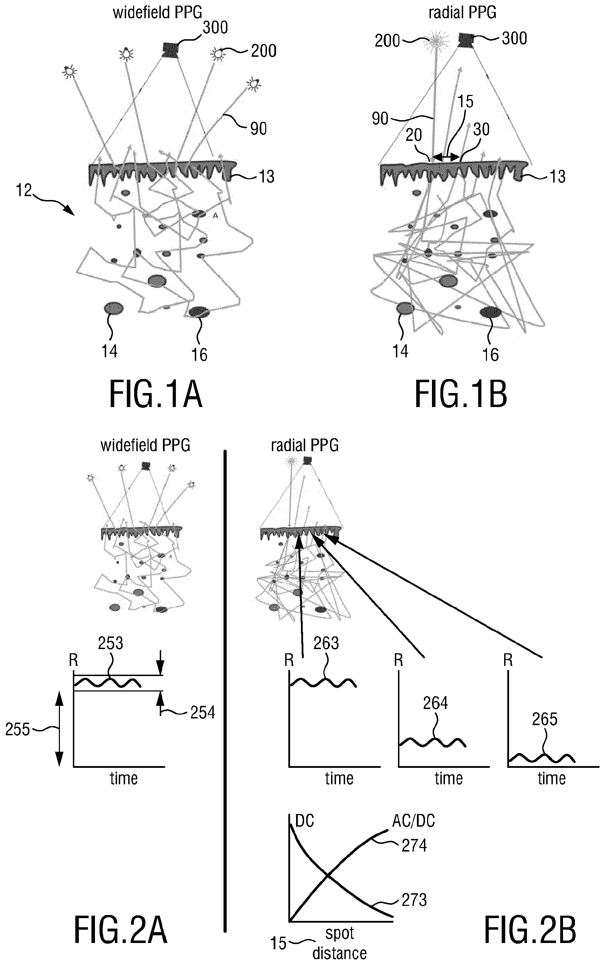

Figs. 1A and 1B show schematic diagrams illustrating the path of electromagnetic radiation 90 through skin region 12 of a subject for widefield PPG (left) and radial PPG (right). Widefield PPG is the mode that is commonly used in camera mode for various years, where the illumination by electromagnetic radiation 90 is homogenously distributed across the skin region 12 and the PPG signal is measured across that same skin area 12. This mode is shown in Fig. 1A. Exemplarily, the electromagnetic radiation 90 is emitted by multiple illumination sources 200, travels through the epidermis 13 of the skin region 12 into deeper skin layers with various venules 14 and arterioles 16 before exiting the skin region 12 and getting detected by a detection unit 300, such as a camera. The detected electromagnetic radiation and the derived PPG signal is an average of all the electromagnetic radiation 90 detected by the camera 300.

Fig. 1B shows a radial PPG mode. The simplest form of contactless radial PPG is the use of just one spot of light injection as shown in Fig. 1B. This may be achieved by use of a laser configured to emit electromagnetic radiation 90 located, e.g., in the red or infrared spectral range. Radial PPG is in principle quite similar to conventional contact probe PPG measurements. The skin region 12 is illuminated by a spot (such as a circle, a stripe, a dot, an ellipse, etc.) and the PPG signal is measured several millimeters or centimeters away from that illumination spot 20 on the skin region 12. The spot distance 15 between the illumination spot 20 on the skin region 12 and the measurement spot 30 from which the PPG signal is measured is exemplarily illustrated for one reflected beam in Fig. 1B. Hence, the radial PPG signal is in general a signal which depends on the spot distance 15. To be more concrete, the electromagnetic radiation reflected from the skin region 12 of the subject is reflected from said skin region 12 by entering into the skin region 12 through the epidermis 13 at the illumination spot 20, getting reflected from constituents of the skin region 12 and exiting the skin region 12 through the epidermis 13 at a measurement spot 30, which is located at a spot distance 15 away from the illumination spot 20. In other words, the electromagnetic radiation detected by the detection unit 300 is scattered back from the skin region 12 and collects pulsatile information from the different venules 14 and arterioles 16 located in the skin region 12 below the epidermis 13.

It will be shown below that the present invention combines according to some embodiments PPG signals obtained from these two measurement modes, i.e., from radial PPG and widefield PPG.

Figs. 2A and 2B show schematic diagrams illustrating the detected reflected light of widefield PPG and radial PPG. The schematic diagrams on the top of Figs. 2A and 2B are the same that have already been discussed with reference to previous Figs. 1A and 1B.

Fig. 2A shows (on the bottom) a diagram illustrating the detected reflected light of a widefield PPG setup. For this purpose, the axis of ordinate illustrates the reflected detected light (indicated by "R") and the axis of abscissae illustrates the measurement time. The curve 253 illustrates the reflected detected light, which comprises a DC component 255 and an AC component 254. The AC component 254 represents the pulsatile components of optical absorption originating from the pulsatile arterial blood and the DC component 255 represents the non-pulsatile components containing contribution from non-pulsatile arterial blood, venous blood, and other tissues.

Fig. 2B shows in the middle row three different diagrams illustrating the detected reflected light of a radial PPG setup. For this purpose, the axes of ordinate illustrate the reflected detected light and the axes of abscissae illustrate the measurement time. It becomes apparent from the three curves 263, 264, 265 that the larger the spot distance 15 between the illumination spot 20 and the respective measurement spot 30 (cf. description with reference to Figs. 1B), the smaller the DC component of the reflected detected light. This is schematically illustrated by the offsets of the curves 263, 264, 265, respectively. The reason for this is that more light or electromagnetic radiation 90 is absorbed by the skin region 12, if the light travels through skin by a longer path.

These dependencies are also illustrated in the lowermost diagram at the bottom of Fig. 2B. This diagram illustrates the AC or DC component of the reflected detected light on the axis of ordinate and the spot distance 15 on the axis of abscissae. The first curve 273 illustrates the DC component of the reflected detected light and the second curve 274 illustrates the AC/DC component (relative PPG amplitude) of the reflected detected light. The already discussed trend becomes apparent. It becomes clear that the larger the spot distance 15, the larger the ratio AC/DC (the larger the relative pulsatile component).

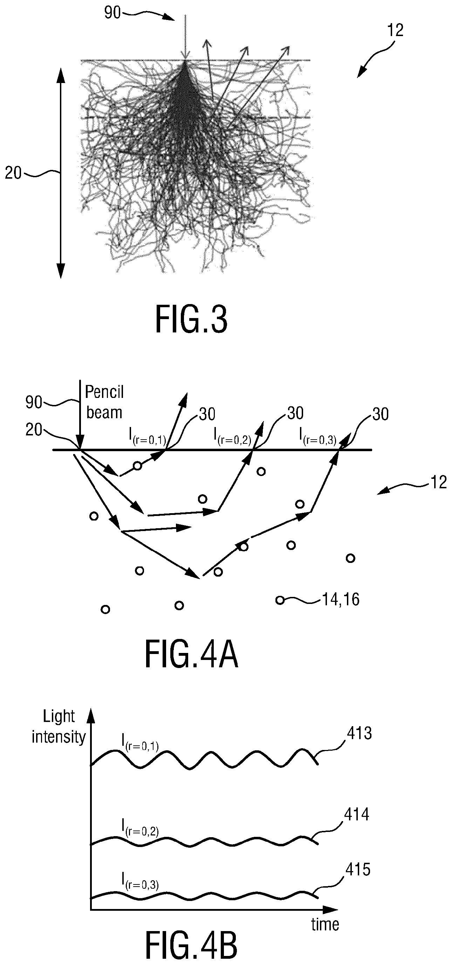

Fig. 3 shows a schematic diagram of Monte Carlo simulations of the path of light through the skin region 12 of a subject for radial PPG. The light distribution inside the skin region 12 is visualized to illustrate the different penetration depths 20, which depicts how depth the incoming electromagnetic radiation 90 penetrates into the skin region 12 before being scattered back from venules and arterioles.

Figs. 4A and 4B show further schematic diagrams illustrating even more clearly the path of electromagnetic radiation 90 through skin region 12 of a subject for radial PPG. As shown in Fig. 4A, the electromagnetic radiation 90 enters the skin region 12 at an illumination spot 20, is reflected from constituents of the skin region 12 (mainly venules 14 and arterioles 16) and exits the skin region 12 at measurement spots 30 distanced away from the illumination spot 20. Exemplarily, three different measurement spots are shown in Fig. 4A. These three different measurement spots 30 exemplarily have a distance of 0.1 mm, 0.2 mm and 0.3 mm to the illumination spot 20 at which a pencil beam (e.g., a laser beam) hits the skin surface.

The light intensity of the detected electromagnetic radiation 90 derived from the three different measurement spots 30 is illustrated in dependence on the measurement time in Fig. 4B. The AC and DC components of the three different curves 413, 414 and 415 clearly depend on how long the electromagnetic radiation 90 has travelled through tissue. It becomes apparent that the longer the light has travelled through skin region 12 with pulsatile volumetric absorbers, the larger the modulation depth of its intensity.

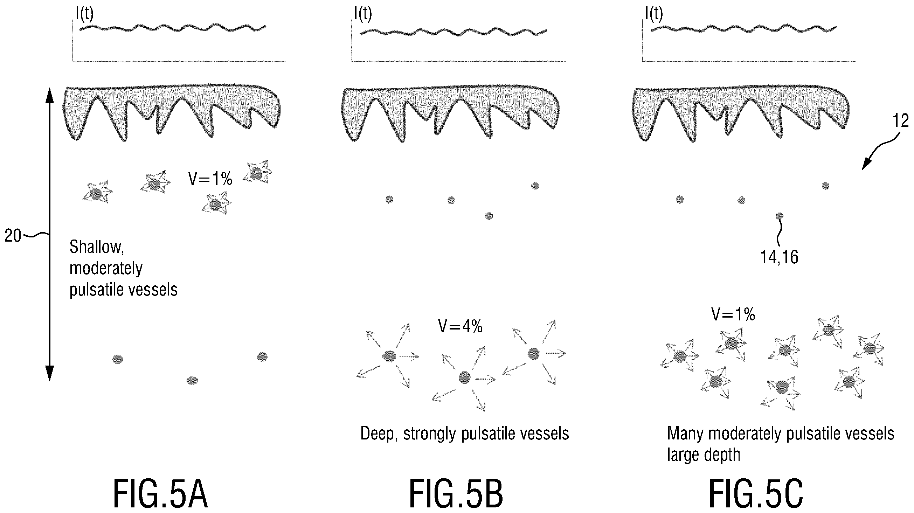

Fig. 5 shows a schematic diagram illustrating ambiguity problems arising for conventional PPG. While PPG images do give an indication of blood perfusion as illustrated in Figs. 4A and 4B, the actual amplitude (i.e., the ratio of the AC and DC component, also referred to as normalized pulsatility) is difficult to interpret, because it is the integral of the PPG modulation resulting from reflected light from different skin depths 20. Hence, superficial vessels with a very small pulsatility as shown in the diagram on the left may give the same PPG signal strength as deeper vessels with stronger pulsatility as shown on the right of Fig. 5. Thus, different locations of strongly or less strongly pulsatile components in the skin region 12 as shown in Fig. 5 may result in exact the same PPG signal strength as illustrated by the curves I(t) in the upper diagrams.

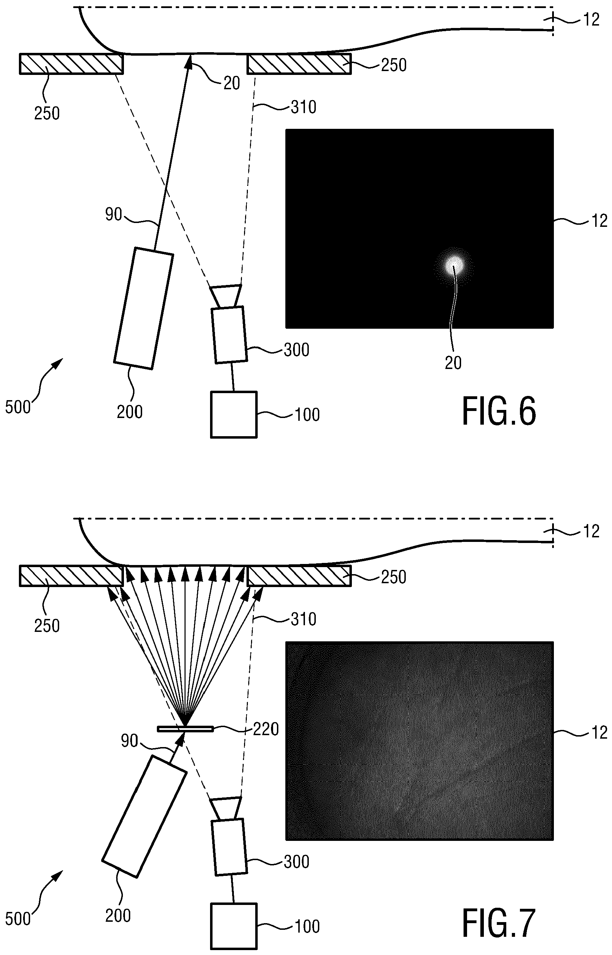

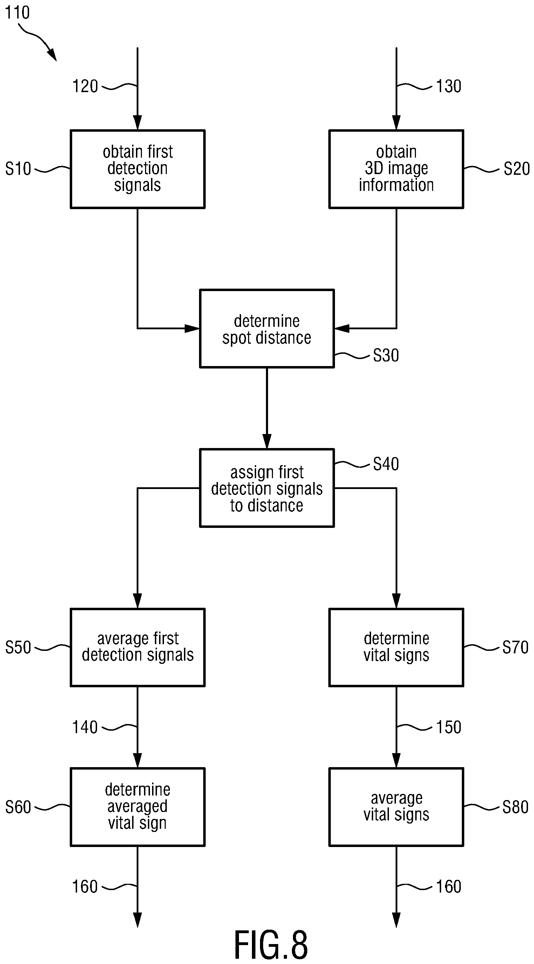

Fig. 6 shows a system 500 for determining a vital sign of a subject according to the present invention. The system 500 comprises at least an illumination unit 200, a detection unit 300 and a device 100 for determining a vital sign of a subject. A more detailed explanation of said device 100 will be given below with reference to Fig. 8.

The illumination unit 200 is configured to emit electromagnetic radiation 90 to illuminate a skin region 12 of a subject. Preferably, said illumination unit 200 is configured to emit a controllable narrow beam of electromagnetic radiation 90, which is preferably located in the visible or infrared spectral range. Infrared radiation is not visible and thus preferred in some clinical settings in order to not disturb the patient.

According to the embodiment shown in Fig. 6, the system 500 may optionally further comprise a support 250 for limiting the skin region 12 of the subject to a limited skin area to be measured. The support 250 may be placed on the skin of the subject as shown in Fig. 6 and is preferably made of a material that is non-transparent for the incoming electromagnetic radiation 90. Still preferably, the skin region 12 used for measurement is thus limited to an area which comprises a skin region 12 with a homogenous surface along the area to be measured.

The inlet in Fig. 6 shows a picture of the illumination profile of an illumination spot 20 on the skin region 12. It shall be understood that one illumination spot 20 is only exemplarily. The illumination unit 20 may also be configured to project an illumination pattern, i.e., multiple illumination spots on the skin region 12 of the subject.

Fig. 7 shows a modified embodiment of the system shown in Fig. 6 in order to illuminate a skin region homogenously. For said purpose, the system 500 may comprise a diffuser 220. Said diffuser 220 is configured to diffuse the controllable narrow beam of electromagnetic radiation 90 emitted by the illumination unit 200 to generate a homogenous illumination profile and/or structured light on the skin region 12 of the subject. Hence, the configuration shown in Fig. 7 allows to measure widefield PPG.

It shall be understood that it may also be an option to use separate illumination units for measuring widefield PPG with a homogenous illumination and radial PPG with a spot illumination. The embodiment shown in Fig. 7, however, provides advantages as a first measurement may be performed for measuring radial PPG by not placing the diffuser into the path of the emitted electromagnetic radiation 90 and a second measurement may be performed for measuring widefield PPG by placing the diffuser into the path of the emitted electromagnetic radiation 90. It will be explained below that the PPG signals obtained for radial PPG and widefield PPG are combined according to some embodiments of the present invention.

Fig. 8 shows a flowchart illustrating a method to be executed by the device 100 for determining a vital sign of a subject according to the present invention.

The device 100 comprises a processing unit 110, which, in a step S10, obtains multiple first detection signals 120 derived from electromagnetic radiation 90 from multiple measurement spots on a skin region 12 of the subject illuminated by one or more illumination spots 20, wherein the electromagnetic radiation 90 enters the skin region 12 at the one or more illumination spots 20 and exits the skin region 12 at the multiple measurement spots 30. This has already been discussed with reference to Fig. 4A, where exemplarily one illumination spot 20 is shown, at which the electromagnetic radiation 90 enters the skin region 12.

In a step S20, the processing unit 110 obtains 3D image information 130 of the skin region 12. The processing unit may either obtain the 3D image information from an external sensor or may determine the 3D image information of the skin region 12 from the multiple first detection signals, in particular in case the skin region 12 is illuminated by multiple illumination spots 20.

In a step S30, the processing unit 110 determines for the multiple first detection signals 120 the respective spot distance 15 between the respective measurement spot 30 and an illumination spot 20 based on the obtained 3D image information 130 of the skin region 12. In a step S40, the processing unit 110 assigns the multiple first detection signals 120 to the respective spot distance 15 and to the illumination spot 20 from which the respective spot distance 15 is determined. Subsequently, the processing unit 110 either executes i) steps S50 and S60, or ii) steps S70 and S80. Thus, in a step S50, the processing unit 110 may average the first detection signals 120 assigned to the same illumination spot 20 and to a respective spot distance 15 smaller than a threshold distance value to obtain an averaged first detection signal 140, and in a step S60, determine an averaged vital sign 160 from said averaged first detection signal. Alternatively, the processing unit 110 may, in a step S70, determine vital signs 150 from the first detection signals 120 assigned to the same illumination spot 20 and to a respective spot distance 15 smaller than a threshold distance value, and in a step S80, average said vital signs 150 to obtain an averaged vital sign 160. It shall be understood that these two options are equivalent. Hence, the averaging may either take place before or after determining a vital sign.

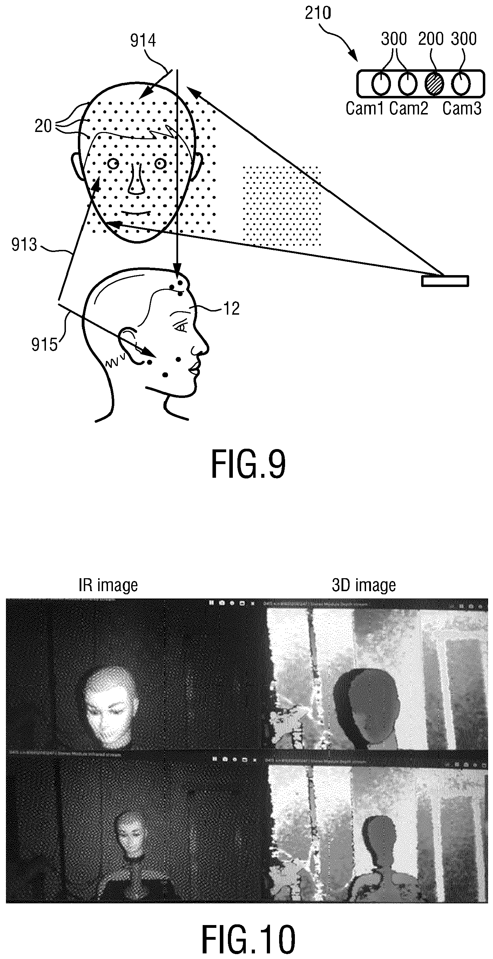

Fig. 9 shows a schematic diagram illustrating problems arising from radial PPG without having knowledge about the 3D structure of the skin region. Exemplarily, a module 210 is used which comprises an illumination unit 200 for emitting electromagnetic radiation to illuminate a skin region 12 (e.g., the head) of a subject by multiple illumination spots 20. Further, the module 210 comprises three cameras 300 configured to record images of the detected electromagnetic radiation 90 reflected from the skin region 12 illuminated by the multiple illumination spots 20. It shall be understood that this is only exemplarily. In general, at least one camera 300 and at least one illumination unit 200 is needed, which, however, do also not have to be integrally formed in one module as exemplarily illustrated in Fig. 9.

The distance between the illumination spots 20 on the forehead of the subject (indicated by arrow 914) may be easily determined by the distance to the module 210 to the subject, i.e., by use of time-of-flight, TOF, for example. For said purpose, the system as shown in Figs. 6 and 7 may further comprise, e.g., a TOF sensor configured to determine 3D image information of the skin region 12. Another option may, for example, to use stereo depth sensors for said purpose, where distance information is obtained by stereo vision.

In difference to the spots 20 on the forehead of the subject, a problem arises from the distance between the illumination spots 20 on the cheeks (as indicated by arrows 913 and 915). Said distances are larger due to the angle of skin with respect to the illumination direction. Thus, without having knowledge about the 3D structure of the skin area 12 it is not possible to quantitatively interpret the PPG signals since only a projected distance may be determined from a color image recorded by the camera 300. This leads to the drawback that the radial PPG signals are wrongly assigned to the projected distance instead of the actual distance.

The present invention is able to overcome said problems by using 3D depth image information. This allows determining the actual distances of the illumination spots 20 on the surface of the skin area 20, and not only a projected distance. Another advantage arising from the present invention is that by having knowledge about the 3D structure of the skin region, multiple measurement spots from a homogenous skin area can be averaged to increase the PPG signal strength.

Fig. 10 shows an infrared image (left) and a 3D depth image (right) of a mannequin. It is a well-known technique to illuminate objects by a spot pattern to extract depth information. For said purpose, an infrared pattern is projected onto an object (or a mannequin as shown in Fig. 10) and, for example, one or more standard CMOS sensors of a camera image the reflected light. One can then estimate depth for each pixel using knowledge of the projected light pattern or by using disparity estimation (when using the images of at least two CMOS sensors). This may, e.g., be achieved by using the module 210 shown in Fig. 9, where an infrared pattern is projected onto a subject and used to infer depth using three cameras.

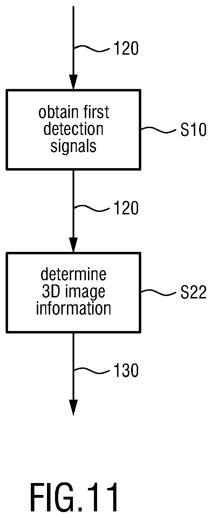

Fig. 11 shows a flowchart illustrating an optional method to be executed by the processor 110 of the claimed device 100. As already explained with reference to Fig. 8, in a step S10, the processor obtains first detection signals derived from electromagnetic radiation 90 from multiple measurement spots on the skin region 20 of the subject. In case the skin region 12 is illuminated by multiple illumination spots 20, the processor may determine, in a step S22, the 3D image information from the multiple first detection signals 120. Hence, the processor may determine this information by its own, without obtaining this 3D information from an external sensor (such as a TOF sensor). This provides the advantage that an external sensor configured to measure the depth information is not needed. The device 100 comprising the processor 110 may thus be a compact device, which may further be portable to be used in various different settings, such as for non-stationary settings and ambulant patients.

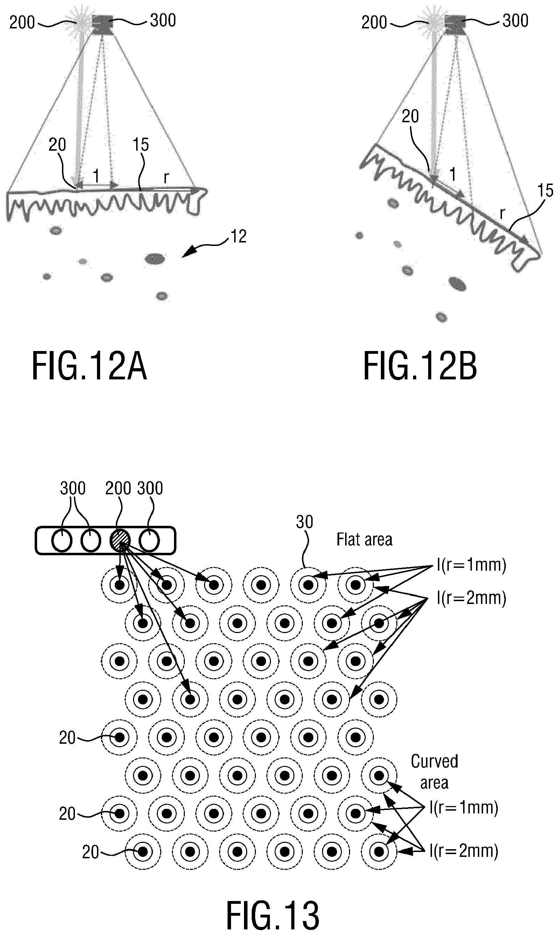

Figs. 12A and 12B show a schematic diagrams illustrating, as Fig. 9, problems arising from conventional radial PPG without having knowledge about the 3D structure of the measured skin region 12. As already mentioned above, radial PPG aims at measuring light intensities as a function of lateral (radial) distance away from the illumination spot 20. Exemplarily, one illumination unit 200 is shown which illuminates the skin region 12 by one illumination spot 20. A camera 300 detects the electromagnetic radiation reflected from the skin region 12, wherein only the reflected light within a field of view, FOV, of the camera 300 is detected.

Fig. 12A illustrates a scenario where the surface of the skin region 12 is perpendicular to the illumination direction of the illumination unit 200. In other words, a normal to a plane defined by the skin surface is parallel to the illumination direction. In this case, distances on the skin region may be easily determined by the distance of the skin region 12 to the camera 300, i.e., by measuring said distance using, for example, TOF technique. Exemplarily, a unit distance and a distance r are shown, while the distance r may be the spot distance 15 between the illumination spot 20 and a measurement spot 30 (as explained above).

Fig. 12B illustrates a scenario where the surface of the skin region is not perpendicular to the illumination direction of the illumination unit 200. In this case, distances on the skin region 12 cannot easily be determined without having knowledge about the 3D structure of the skin region 12. This is illustrated again by the same unit distance and the distance r. The lengths of the arrows indicating said unit distance and the distance r (or the spot distance 15) are smaller than the lengths of the arrows in Fig. 12A. This illustrates that there is a need for correcting for projection if the skin is not perpendicular to the illumination direction of the illumination unit 200. Otherwise the measured radial PPG signal is assigned to a projected (smaller) distance, rather than to the actual (correct) distance.

Fig. 13 shows a schematic diagram illustrating an area illuminated by a spot pattern. The area may be the skin region illuminated for PPG measurements as described above.

The same module 210 as already explained with reference to Fig. 9 may be used here, which comprises an illumination unit 200 and three cameras 300. The illumination unit 200 illuminates the skin area 12 by multiple illumination spots 20. Exemplarily, the illumination spots 20 have the shape of dots. However, it shall be understood that the illumination spots 20 may also have the shape of a line, an ellipse, circles, etc.

Each dashed circle around an illumination spot 20 represents possible measurement spots 30, which all have the same spot distance 15 to the respective illumination spot 20. Thus, the measurement spots on the inner dashed circles may exemplarily have a spot distance of 1 mm to the respective illumination spot 20. The measurement spots on the outer dashed circles may exemplarily have a spot distance of 2 mm to the respective illumination spot. The first detection signals derived from electromagnetic radiation from multiple measurement spots on a skin region of the subject illuminated by multiple illumination spots may thus be abbreviated by I(r=1 mm) and I(r=2 mm), respectively, as shown in Fig. 13. If the area is not flat, but curved, i.e., if the area is not perpendicular to the illumination direction of the illumination unit 200, the dashed circles transform into dashed ellipses, which represents the problem discussed already with reference to Figs. 9 and 12. Thus, the dashed circles transform into ellipses for a curved area as shown in the lower part of the area shown in Fig. 13.

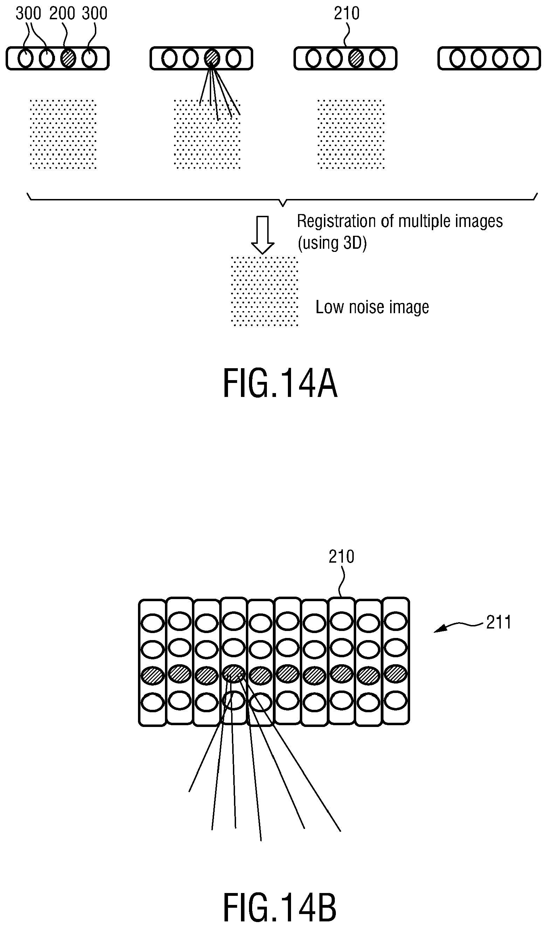

Figs. 14A and 14B show a schematic diagrams illustrating the use of multiple modules 210 each comprising an illumination unit 200 and three cameras 300. A powerful technique is to reduce noise of the measured first detection signals by combining multiple modules 210 and use the spot pattern to register the camera images of these. As shown in Fig. 14A, one of the modules 210 may be used to project the illumination pattern (as indicated by the arrows), while the other modules are used for imaging only, i.e., to record the electromagnetic radiation reflected from the area (such as the measured skin region). Fig. 14B shows a schematic diagram illustrating the use of 10 modules as a module array 211. A vertical placement allows for placing 10 (or even more) modules 210 at a small distance with limited parallax. Thus, noise may be further reduced by averaging the signals obtained from a plurality of modules 210.

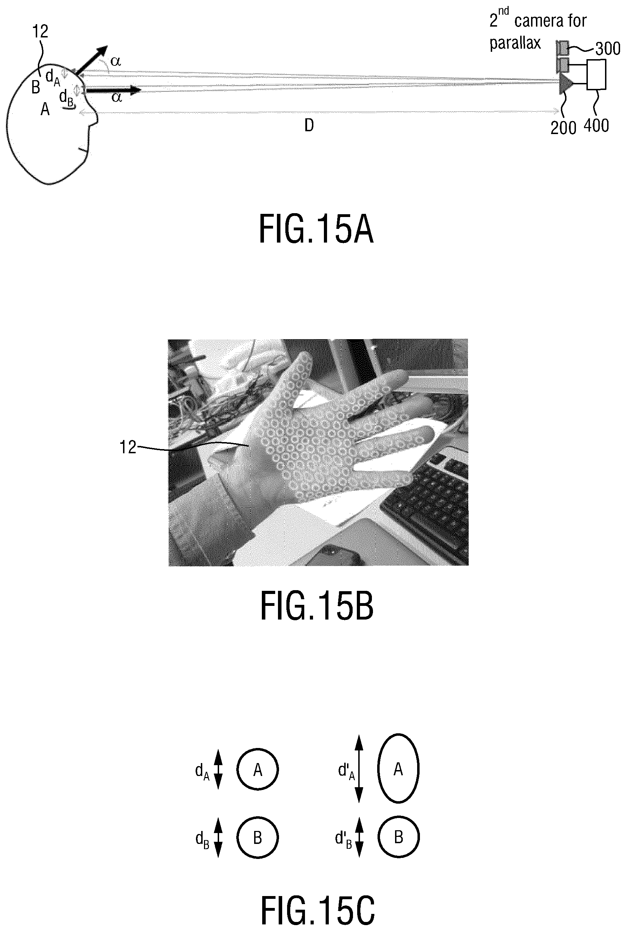

Fig. 15 shows schematic diagrams illustrating the projection of a pattern of circles on a skin region of a subject. The skin region 12 may either be on the head of the subject as shown in Fig. 12A or on the hand as shown in Fig. 12B. However, it shall be understood that these two skin regions are only exemplarily. It shall be noted that measuring PPG signals from the forehead of the cheek of a subject as illustrated in Fig. 15A is well-known to give decent PPG signal strengths, while measuring PPG signals on other anatomical locations, such as on the arms, is known to give much lower PPG signal strengths.

As shown in Fig. 15A, an illumination unit 200 is used to project the pattern of circles onto the skin region 12 of the subject, which is located at a distance D away from the illumination unit 200. An image of such a pattern projected onto a hand of the subject is shown in Fig. 15B. This image may be recorded by the camera 300 shown in Fig. 15A, which may be located directly adjacent to the illumination unit 200. A second camera may be used as well to correct for parallax.

It becomes apparent that the shape of the projected pattern depends on the angle α of the skin region 12 with respect to the illumination direction. If the angle is zero, i.e., if the skin region 12 is perpendicular to the illumination direction, the projected pattern on the skin region 12 comprises circles, while if the angle is different from zero, i.e., if the skin region 12 is not perpendicular to the illumination direction, the projected pattern comprises ellipses. In other words, the projected pattern of circles appears as ellipses on the skin region 12 due to the non-zero angle α.

Fig. 15C shows a schematic diagram illustrating the shape of the projected circles on different locations on the skin region 12 indicated by "A" and "B" in Fig. 15A, where location "B" is located further away from the nose of the subject than location "A". The angle α is much larger for location "B", while said angle is almost zero for location "A". Hence, the diameter d_{A} of the projected circle onto location "A" is strongly modified as shown in Fig. 15C. The projected circle appears as an ellipse with a modified diameter d_{A}'= d_{A}/cos(α). The diameter d_{B} of the projected circle onto location "B" is hardly modified (d_{B} ≈ d_{B}'). Hence, the circle projected onto location "B" appears as a circle and does not transform into an ellipse. This can also be seen in Fig. 15B, where the projected circles on locations with a large angle α, such as the insides of the fingers, appear as ellipses. It shall be understood that the modifications of the projected circles into ellipses does not only depend on the diameters of the projected circles, and angle α, but also on the distance D between the illumination unit 200 and the akin region 12 of the subject.

It will be shown later that the PPG signal strength drop off increases for larger spot distances (i.e., large distances between an illumination of the spot pattern and the respective measurement spot). Even with a low noise/high sensitivity camera this is a very challenging dynamic range. By using circular illumination patterns, much more camera pixels are within a certain dynamic range without losing the ability to measure a radial PPG signal.

Hence, to only use a pattern of circles there are various possibilities. One option is to discard all illumination spots with a strong ellipsoidal illumination (e.g., for angles α > 10°) for further processing of PPG signals. It may thus be an option to discard the signals derived from an image area in the recorded image of the camera 200 which comprises many ellipsoidal illuminations (such as location "A" in Fig. 15A).

Another option is to modify the illumination of the illumination spots on location "A" such that the actual illumination is a circle again. This may be done as follows: A control unit 400 may be connected to the illumination 200 and the camera 300 as shown in Fig. 15A. Said control unit 400 may receive 3D image information of the skin region 12 and a color image of the illuminated skin region 12 recorded by the camera 300 being a color camera. Based on this, the control unit 400 may control the shape of the illumination spots (the pattern of circles) emitted by the illumination unit 200 to emit illumination spots having the shape of such an ellipse that the illumination spots appear as circular spots again (in the image recorded by the camera 300).

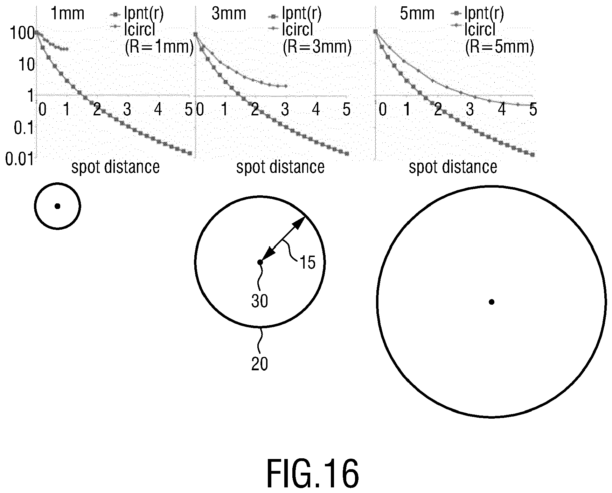

Fig. 16 shows a schematic diagram illustrating a comparison of an illumination by a dot pattern and an illumination by a circular pattern. The upper three diagrams illustrate respectively the DC component of the reflected intensity in dependence on the spot distance 15. In case of a circular spot pattern, the spot distance is again determined by the (smallest) distance of the respective measurement 30 spot to an illumination spot 20. The upper curves in the respective diagrams illustrate the reflected intensity derived for a circular spot pattern, while the lower curves illustrate the reflected intensity derived for a dot pattern. The diagram on the left illustrates the reflected intensity derived for a circular spot pattern, where the projected spots have a diameter of 1 mm. The middle diagram and the right diagram refer to illuminations with circular diameters of 3 mm and 5 mm, respectively (cf. sketches of the respective circles below). As in all three diagrams the circular spot pattern curves comprise larger intensity values than the dot pattern curves, the DC intensity drop-off inside an illumination circle is much less than for a dot. This illustrates that by using circular illumination patterns, much more camera pixels are within a certain dynamic range without losing the ability to measure a radial PPG signal.

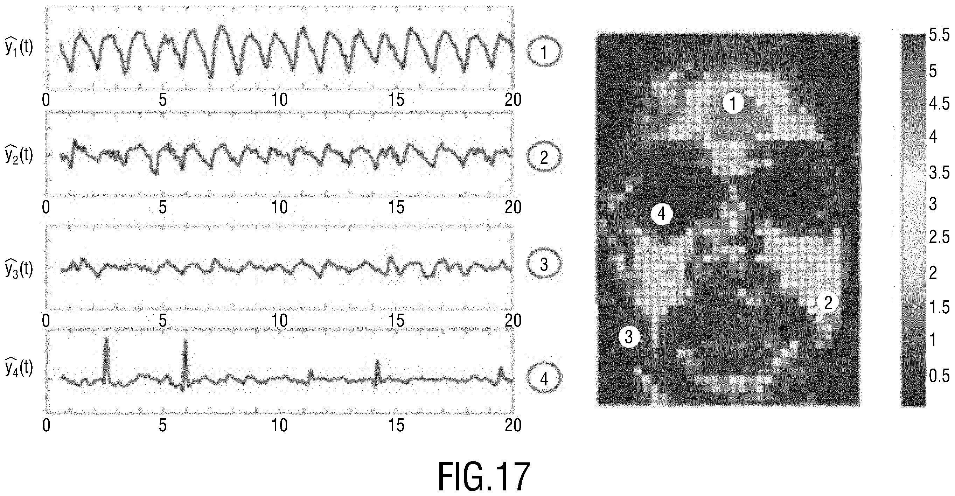

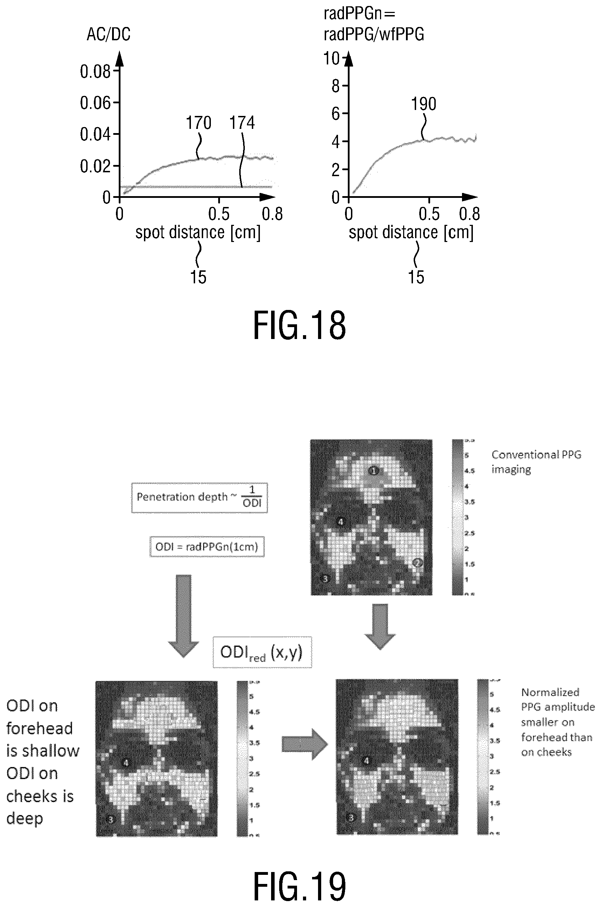

Fig. 17 shows a schematic diagram illustrating locally measured PPG amplitudes. The color map image on the right displays the amplitude of locally measured PPG amplitudes and the diagrams on the left show time signals of the respective PPG amplitudes measured at four different locations on the skin area 12 indicated by numbers. The color image is difficult to interpret for clinical use, because the signal displayed is a convolution of PPG modulations stemming from different skin depths as already explained with reference to Fig. 5. Superficial vessels with a very small pulsatility may, e.g., give the same PPG signal strength as deeper vessels with a stronger pulsatility. Hence, it is not known from the color map shown in Fig. 17 whether the large signal in face region indicated by number "1" is due to relatively shallow arterioles, or whether there is a higher density of arterioles since the overall signal is a weighted sum of pulsatile components at different depths. This ambiguity may also be one of the reasons why PPG imaging experiences slow acceptance in clinical practice. When a clinician interprets the color map, it is difficult to explain what they actually refer to since many parameters are involved, such as the optical depth of the pulsatile vessels, strength of the volumetric pulsatility, density of pulsatile vessels and absorption coefficient of the blood at the wavelength used. With reference to the subsequent Fig. 18 it is explained how to define a signal which indicates the optical depth of the pulsatile vessels and may thus be used for less ambiguous PPG imaging.

Fig. 18 shows a processing of radial PPG signals and widefield PPG signals to overcome problems arising from conventional PPG. As explained above, the first detection signals refer to radial PPG signals and the processing unit of the claimed device may be configured to derive a first detection function 170 from the first detection signals assigned to the same illumination spot 20. The first detection function 170 is a function of the spot distance as shown in Fig. 18 and may depict the relative pulsatile component (AC/DC) of the first detection signals.

The second detection signal, as explained above, refers to a widefield PPG signal. The processing unit of the claimed device may further be configured to derive a second detection function 174 from the second detection signal. As the second detection function refers to widefield PPG and a homogenous illumination, the second detection signal, which may also depict the relative pulsatile component (AC/DC) of the second detection signal, is independent from the spot distance.

In a next step, the processing unit may be configured to determine a normalized signal 190 by calculating a ratio of the first detection function 170 derived from the first detection signals and the second detection function 174 derived from the second detection signal. This normalized signal 190 which refers to a ratio of radial PPG signal amplitudes and widefield PPG signal amplitudes, can be used to give an index, which depends on the spot distance 15. This index may be referred to as "Optical Depth Index" or "ODI" and represents an estimate of the relative optical depth of the source of the PPG. In other words, said normalized signal 190 or said ODI gives an estimate for the dominant depth of the pulsatile vessels Thus, this ODI allows obtaining information about the dominant depth of pulsatile vessels at which the electromagnetic radiation is scattered back.

Fig. 19 shows a schematic diagram illustrating a process of obtaining improved PPG color maps. The uppermost color map represents a typical color map derived from conventional PPG imaging, where the color represents the strength of the PPG amplitude. Said color map is, as already mentioned above, difficult to interpret as it is not known from said color map whether the large signal in the face region indicated by number "1" is due to relatively shallow arterioles or whether there is a higher density of arterioles since the overall signal is a weighted sum of pulsatile components at different depths. However, as explained with reference to Fig. 18, the ratio of radial PPG signals and a widefield PPG signal gives an index, ODI, which allows estimating the penetration depth of the electromagnetic radiation in the skin of the subject. This ODI is, as shown in the right diagram of Fig. 18, dependent on the spot distance 15. For simplifications, a constant ODI value may thus be defined by setting, e.g., the spot distance to 1 cm. This allows determining a color map for each skin pixel with a discrete ODI value. This color map is shown in the bottom left of Fig. 19. Said color map illustrates that the ODI on the forehead is typically low, while the ODI on the cheek is typically large. The color map on the bottom right of Fig. 19 is a sum of the ODI color map and the color map obtained from conventional PPG imaging without having information about the optical depth of the pulsatile layers. This color map is thus supposed to give more accurate results and improves PPG imaging as the PPG signal strengths is weighted with the ODI.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (100) for determining a vital sign of a subject, comprising a processing unit (110) configured to:
- obtain multiple first detection signals (120) derived from electromagnetic radiation (90) from multiple measurement spots (30) on a skin region (12) of the subject illuminated by one or more illumination spots (20), wherein the electromagnetic radiation (90) enters the skin region (12) at the one or more illumination spots (20) and exits the skin region (12) at the multiple measurement spots (30),
- obtain a 3D image information (130) of the skin region (12),
- determine for the multiple first detection signals (120) the respective spot distance (15) between the respective measurement spot (30) and an illumination spot (20) based on the obtained 3D image information (130) of the skin region (12),
- assign the multiple first detection signals (120) to their respective spot distance (15) and to the illumination spot (20) from which the respective spot distance (15) is determined, and
- average the first detection signals (120) assigned to the same illumination spot (20) and to a respective spot distance (15) smaller than a threshold distance value to obtain an averaged first detection signal (140), and determine an averaged vital sign (160) from said averaged first detection signal (140), or
- determine vital signs (150) from the first detection signals (120) assigned to the same illumination spot (20) and to a respective spot distance (15) smaller than a threshold distance value, and average said vital signs (150) to obtain an averaged vital sign (160).

2. Device (100) according to claim 1, wherein the processing unit (110) is further configured to derive a first detection function (170) from the first detection signals (120) assigned to the same illumination spot (20), wherein the first detection function (170) is a function of the spot distance (15).

3. Device according to any one of the preceding claims,
wherein the processing unit (110) is configured to determine the 3D image information (130) of the skin region (12) from the multiple first detection signals (120) in case the skin region (12) is illuminated by multiple illumination spots (20).

4. Device according to any one of the preceding claims,
wherein, in the case of multiple illumination spots (20), the processing unit (110) is configured to determine for the multiple first detection signals (120) the respective spot distance (15) between the respective measurement spot (30) and the illumination spot (20) which is the closest illumination spot to the respective measurement spot (30) in relation to the other illumination spots (20).

5. Device according to any one of the preceding claims,
wherein the processing unit (110) is configured to:
- obtain a second detection signal (180) derived from a spatial integral of all the electromagnetic radiation (90) reflected from the skin region (12) of the subject illuminated by one or more illumination spots (20) or derived from electromagnetic radiation (90) reflected from or transmitted through skin region (12) of the subject illuminated by a homogenous illumination, and
- determine one or more normalized signals (190), wherein a normalized signal (190) is calculated by calculating a ratio of a first detection signal (120) of the multiple first detection signals and the second detection signal (180).

6. Device according to any one of the preceding claims,
wherein the multiple first detection signals (120) and the second detection signal (180) are derived from electromagnetic radiation (90) with the same wavelength.

7. System (500) for determining a vital sign of a subject, said system (500) comprising:
- an illumination unit (200) configured to emit electromagnetic radiation (90) to illuminate a skin region of the subject by one or more illumination spots (20),
- a camera (300) configured to record an image of the detected electromagnetic radiation (90) reflected from the skin region (12) illuminated by one or more illumination spots (20) and to derive multiple first detection signals from the recorded images, and
- a device (100) for determining an averaged vital sign (160) of a subject according to any one of the preceding claims.

8. System according to claim 7,
wherein the one or more illumination spots (20) have the shape of any one of a circle, ellipse, dot or stripe.

9. System according to any one of claims 7 or 8,
further comprising a time-of-flight, TOF, sensor configured to determine 3D image information (130) of the skin region (12) from the detected electromagnetic radiation (90) reflected from the skin region illuminated by one or more illumination spots (20) and/or a stereo depth sensor configured to determine 3D image information (130) of the skin region by stereo vision.

10. System according to any one of claim 7 to 9, further comprising
a control unit (400),
wherein, in the case where the illumination unit (200) is configured to emit electromagnetic radiation (90) by one or more illumination spots (20) having the shape of a circle or an ellipse, the control unit (400) is configured to receive 3D image information (130) of the skin region (12) and an color image of the illuminated skin region (12) recorded by the camera (300) being a color camera, to control the shape of the one or more illumination spots (20) emitted by the illumination unit (200) based on said received 3D image information (130) and said received color image to emit one or more illumination spots (20) having the shape of such an ellipse that the one or more illumination spots (20) have the shape of a circle on the skin region (12) in the color image recorded by the color camera (300).

11. System according to claims 9 and 10,
wherein the control unit (400) is configured to receive the 3D image information (130) from the processing unit (110) of the device (100) or from the TOF sensor or from the stereo depth sensor.

12. System according to any one of claims 7 to 11,
wherein the camera (300) comprises a plurality of detection elements, in particular an array of photo diodes, a CCD array or a CMOS array.

13. Method for determining a vital sign of a subject, said method comprising the steps of:
- obtaining multiple first detection signals (120) derived from electromagnetic radiation (90) from multiple measurement spots (30) on a skin region (12) of the subject illuminated by one or more illumination spots (20), wherein the electromagnetic radiation (90) enters the skin region (12) at the one or more illumination spots (20) and exits the skin region (12) at the multiple measurement spots (30),
- obtaining a 3D image information (130) of the skin region (12),
- determining for the multiple first detection signals (120) the respective spot distance (15) between the respective measurement spot (30) and an illumination spot (20) based on the obtained 3D image information (130) of the skin region (12),
- assigning the multiple first detection signals (120) to their respective spot distance (15) and to the illumination spot (20) from which the respective spot distance (15) is determined, and
- averaging the first detection signals (120) assigned to the same illumination spot (20) and to a respective spot distance (15) smaller than a threshold distance value to obtain an averaged first detection signal (140), and determine an averaged vital sign (160) from said averaged first detection signal (140), or
- determining vital signs (150) from the first detection signals (120) assigned to the same illumination spot (20) and to a respective spot distance (15) smaller than a threshold distance value, and average said vital signs (150) to obtain an averaged vital sign (160).

14. Computer program comprising program code means for causing a computer to carry out the steps of the method as claimed in claim 13 when said computer program is carried out on the computer.
